# EUROPEAN PATENT SPECIFICATION

(11) **EP 3 221 297 B1**
(45) Date of publication and mention of the grant of the patent: **22.08.2018**
(21) Application number: 15804597.1
(22) Date of filing: 18.11.2015
(51) Int. Cl.: C07D 223/16

(54) **PROCESS FOR THE SYNTHESIS OF BENZAZEPINE DERIVATIVES**
VERFAHREN ZUR SYNTHESE VON BENZAZEPINDERIVATIVEN
PROCÉDÉ POUR LA SYNTHÈSE DE DÉRIVÉS DE BENZAZÉPINE

(30) Priority: 19.11.2014 HU P1400545
(43) Date of publication of application: 27.09.2017
(73) Proprietor: Richter Gedeon Nyrt., 1103 Budapest (HU)
(72) Inventor: GARADNAY, Sándor, H-1037 Budapest (HU); NEU, József, H-1133 Budapest (HU); SZABÓ, Tamás, H-5830 Battonya (HU)
(74) Representative: Hoffmann Eitle
(86) International application number: PCT/IB2015/058913
(87) International publication number: WO 2016/079684

(56) References cited:
- EP-A1- 0 065 229
- US-A1- 2009 318 682
- PHAKHODEE W ET AL: "A new synthetic approach towards isoquinobenzazepinone and isoindolinobenzazepinone using acid-mediated cyclisation and Heck reaction", TETRAHEDRON, ELSEVIER SCIENCE PUBLISHERS, AMSTERDAM, NL, vol. 65, no. 1, 3 January 2009 (2009-01-03), pages 351-356, XP025712255, ISSN: 0040-4020, DOI: 10.1016/J.TET.2008.10.044 [retrieved on 2008-11-01]
- REIFFEN M ET AL: "SPECIFIC BRADYCARDIC AGENTS. 1. CHEMISTRY, PHARMACOLOGY AND STRUCTURE-ACTIVITY RELATIONSHIPS OF SUBSTITUTED BENZAZEPINONES, A NEW CLASS OF COMPOUNDS EXERTING ANTIISCHEMIC PROPERTIES", JOURNAL OF MEDICINAL CHEMISTRY, AMERICAN CHEMICAL SOCIETY, US, vol. 33, no. 5, 1 May 1990 (1990-05-01), pages 1496-1504, XP002047919, ISSN: 0022-2623, DOI: 10.1021/JM00167A033

## Description

The invention relates to a process for the synthesis of pharmaceutically applicable 3-(3-{[((7S)-3,4-dimethoxybicyclo[4,2,0]octa-1,3,5-trien-7-yl)-methyl]-methylamino}-propyl)-1,3,4,5-tetrahydro-7,8-dimethoxy-2H-3-benzazepin-2-one of formula (I), as well as to the synthesis of one key intermediate of the process, 7,8-dimethoxy-1,3-dihydro-benzazepin-2-one of formula (II). 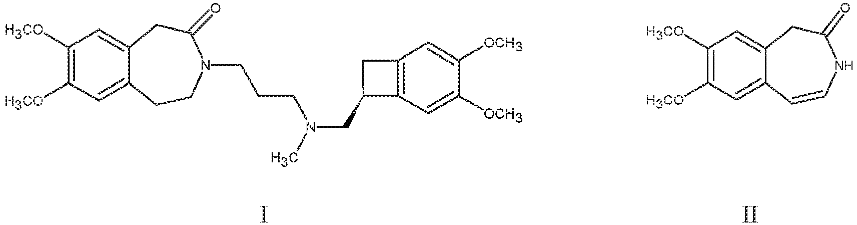 Ivabradin of formula (1) is a known compound, it is the active ingredient of the pharmaceutical composition used for the treatment of the symptoms of permanent angina pectoris and the first synthesis thereof was disclosed in the European patent application No. EP 0 534 859

The first synthesis of 7,8-dimethoxy-1,3-dihydro-benzazepin-2-one of formula (II) was disclosed in the European patent application No. EP 0 285 919 (Figure 1.). In this description 3,4-dimethoxy-phenylacetic acid of formula (III) is reacted with aminoacetaldehyde-dimethyl-acetal in N,N-dimethyl-formamide or dichloromethane using N,N'-dicyclohexyl-carbodiimide (DCC) or N-ethyl-N'-(dimethylamino)-ethylcarbodiimide resulting in N-(2,2-dimethoxyethyl)-2-(3,4-dimethoxyphenyl)-acetamide of formula (V). As an alternative procedure the treatment of 3,4-dimethoxy-phenylacetic acid with thionyl chloride or with phosgene is described resulting in the acid chloride of formula (IV') and this is thereafter reacted with aminoacetaldehyde dimethyl acetal. According to the general description the ring closure of the obtained N-(2,2-dimethoxyethyl)-2-(3,4-dimethoxyphenyl)-acetamide of formula (V) is carried out with a strong acid, such as trifluoromethanesulfonic acid or hydrochloric acid in acetic acid solution. In the examples the 3,4-dimethoxy derivative is not described, therefore the yield of the given reaction steps can not be calculated. In the procedure described in the examples the ring closure of 3-methoxy-phenylacetic acid used as starting material is carried out with acetic acid and concentrated aqueous hydrochloric acid, hence it can be presumed, that no experiments were carried out with trifluoromethanesulfonic acid, otherwise they would have noticed the advantages of that procedure.

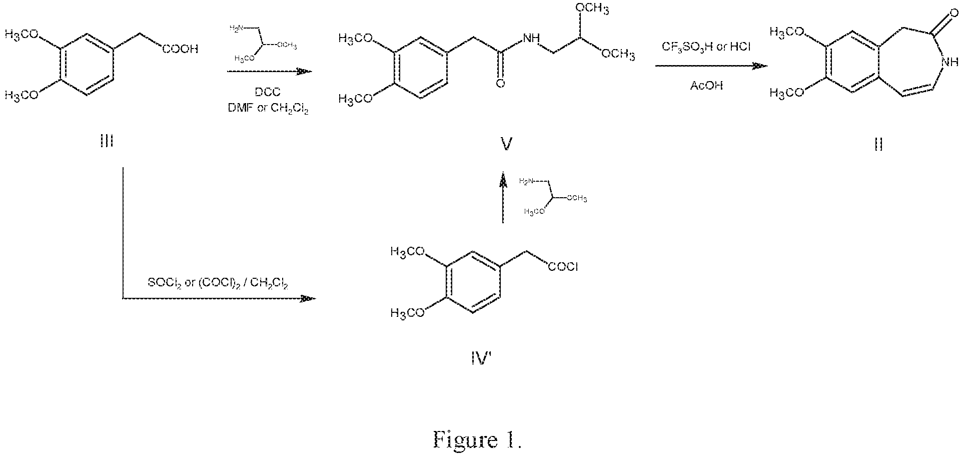

The synthesis of 7,8-dimethoxy-1,3-dihydro-benzazepin-2-one of formula (II) is also described in the Journal of Medicinal Chemistry 1990. 33(5). 1496-1504. (Figure 2). During this procedure 3,4-dimethoxy-fenylacetic acid of formula (III) is reacted with thionyl chloride in dichloromethane, then the obtained 3,4-dimethoxy-phenylacetic acid chloride of formula (IV') is treated with aminoacetaldehyde dimethyl acetal in the pi-esetice of triethylamine in dichloromethane. The so obtained N-(2,2-dimethoxyethyl)-2-(3,4-dimethoxyphenyl)-acetamide of formula (V) is converted into 7,8-dimethoxy-1,3-dihydro-benzazepine-2-one of formula (II) in acetic acid in the presence of concentrated hydrochloric acid.

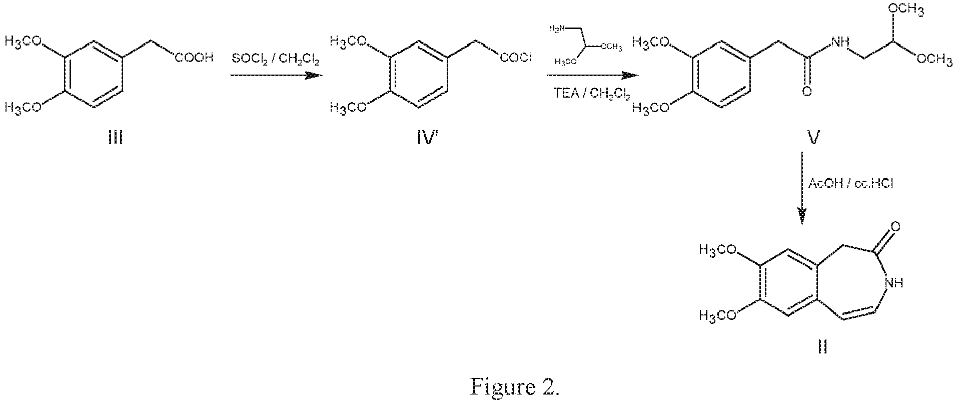

The synthesis of 7,8-dimethoxy-1,3-dihydro-benzazepin-2-one of formula (II) is also described in the patent applications No. US4,584,293, US5,447,928, US2009318682 and WO2010072409.

During this procedure thionyl chloride in dichloromethane is used for the synthesis of the acid chloride, then the latter is reacted with aminoacetaldehyde dimethyl acetal in the presence of triethylamine. The obtained N-(2,2-dimethoxyethyl)-2-(3,4-dimethoxyphenyl)-acetamide of formula (V) is cyclized in acetic acid with concentrated aqueous hydrochloric acid. A common feature of these procedures is the low overall yield, which according to the given data remains in the range of 40-57 %, calculated on the starting 3,4-dimethoxy-phenylacetic acid.

According to the data given in the patent application No. WO2010072409 the overall yield, calculated on the starting 3,4-dimethoxy-phenylacetic acid is only 45.6 %. The purity of the obtained 7,8-dimethoxy-1,3-dihydro-benzazepin-2-one is 98.9 %, determined by HPLC.

According to the patent application No. US4,265,890 the 3,4-dimethoxy-phenylacetic acid is reacted with dicyclohexylcarbodiimide (DCC) in the presence of aminoacetaldehyde dimethyl acetal in dichloromethane. The obtained N-(2,2-dimethoxyethyl)-2-(3,4-dimethoxyphenyl)-acetamide of formula (V) is then converted into 7,8-dimethoxy-1,3-dihydro-benzazepin-2-one of formula (II) in acetic acid in the presence of concentrated aqueous hydrochloric acid. The yield is not given in the description.

The synthesis of 7,8-dimethoxy-1,3-dihydro-benzazepin-2-one of formula (II) is also described in the patent application No. CN10227653. According to the description an active ester is formed from 3,4-dimethoxy-phenylacetic acid with 2,4-dimethoxy-6-chloro-1,3,5-triazine; N,N-dicyclohexylcarbodiimide (DCC); 1-ethyl-3-(3-dimethylaminopropyl)-carbodiimide (EDCI) or N,N-carbonyldiimidazole (CDI) in the presence of a base, such as e.g. N-methyl-morpholine (1.2 molequivalent), triethylamine (1.2 mo1lequivalent), dimethyl-aminopyridine (2.0 molequivalent) or pyridine (2.0 molequivalent). Among the given examples only the use of 1.2 molequivalent of 2,4-dimethoxy-6-chloro-1,3,5-triazine and 1 molequivalent of N,N-dicyclohexylcarbodiimide is described. In the course of their use a yield of 92-93 % is given, but the yield of the reaction mentioned in Example 2, which is carried out with DCC, according to our calculation is only 57 % instead of the given 74 %. Thereafter the obtained N-(2,2-dimethoxyethyl)-2-(3,4-dimethoxyphenyl)-acetamide of formula (V) is converted into 7,8-dimethoxy-1,3-dihydro-benzazepin-2-one of formula (II) in acetic acid in the presence of concentrated aqueous hydrochloric acid. Yield is not given for the ring closure, but for the complete process a yield of 75 % is given.

In the patent application No. CN10227653 an other process is described for the synthesis of N-(2,2-dimethoxyethyl)-2-(3,4-dimethoxyphenyl)-acetamide of formula (V) using thionyl chloride in dichloromethane, when the obtained 3,4-dimethoxy-phenylacetic acid chloride is reacted with aminoacetaldehyde dimethyl acetal. The overall yield calculated on 3,4-dimethoxy-phenylacetic acid without the ring closure is 43.9 %.

According to the above mentioned procedures it is obvious to an expert, that these processes can be economically improved on an industrial scale, as according to the data of the aforementioned patent applications the yield of the 7,8-dimethoxy-1,3-dihydro-benzazepin-2-one of formula (II) remains in most cases below 60 %.

In most cases thionyl chloride is used for the synthesis of 7,8-dimethoxy-1,3-dihydro-benzazepin-2-one of formula (II), the use of which is accompanied with safety hazards, it is environmentally disadvantageous and is unfavorable to use on an industrial scale because of its highly corrosive property. During the synthesis of acid chlorides with thionyl chloride an equivalent amount of hydrogen chloride as well as sulfur dioxide is formed, which on an industrial scale is a serious burden for the environment and difficult to handle from safety point of view. Therefor it is necessary to absorb and neutralize the formed aggressive gases, which further increases the expenses of the synthesis of the product from economical point of view.

The acid chloride, formed in the reaction is unstable under hydrolytic conditions and decomposes easily in the presence of water, this way further decreasing of the yield. During the decomposition dangerous gaseous hydrogen chloride is formed, therefore further technological problems arise during the synthesis, storage and use of the acid chloride on an industrial scale, making further settlings necessary.

When the acid chloride is used for the amidation reaction with aminoacetaldehyde dimethyl acetal it is necessary to apply an acid scavenger for neutralizing the hydrogen chloride formed in the reaction in an equivalent amount to the amide. This is the reason of using triethylamine in the examples mentioned above. Therefore an equivalent amount of triethylamine hydrochloride is formed in the reaction as well, which means the formation of large amount of a side-product on an industrial scale production. The use of lover amount of triethylamine may lead to a lover conversion rate. A further problem may arise if the thionyl chloride is not removed completely, as the remaining traces thereof will consume the triethylamine.

It is also complicated to carry out the amidation reaction with carbodiimides or carbodiimidazoles on an industrial scale. The carbodiimides and carbodiimidazoles are unstable compounds and hydrolyze easily even in the presence of humid air. Their further drawbacks are that the carbamide or imidazole formed as byproduct can often not be removed completely, and the DCC, the EDCI as well as the CDI are strong allergenic substances.

Application of 2,4-dimethoxy-6-chloro-1,3,5-triazine on an industrial scale is disadvantageous mainly from the economical point of view, as the price of 2,4-dimethoxy-6-chloro-1,3,5-triazine is almost tenfold compared to that of DCC and almost fivefold compared to that of 3,4-dimethoxy-phenylacetic acid. One further drawback of its use on an industrial scale is, that 1.2 mol equivalent amount of it has to be applied, increasing the expenses considerably. During the formation of the active ester 1.2-2 mol equivalent of an acid scavenger (N-methyl-morpholine, triethylamine, dimethyl-aminopyridine, pyiridine) has to be used as well, further increasing the expenses. When a large amount of 2,4-dimethoxy-6-chloro-1,3,5-triazine is applied, an equivalent amount of a byproduct (2,4-dimethoxy-6-hydroxy-1,3,5-triazine), and an equivalent amount of hydrochloride salt of the applied base is formed as well, which has to be dealt with in the case of an industrial scale production, and these byproducts can contaminate the obtained product respectively.

In all examples mentioned above dichloromethane was used as solvent for the formation of acid chloride as well as for the amidation reaction, which is a halogenated solvent and its use has to be omitted on an industrial scale. The neutralization of the high amount of solvent used in the reaction represents a further cost-rising factor.

According to the above descriptions concentrated acid solutions are used for the ring-closing reactions, which contain water as well. However in the presence of water the acetal functional group of N-(2,2-dimethoxyethyl)-2-(3,4-dimethoxyphenyl)-acetamide of formula (V) undergoes a hydrolytic decomposition decreasing thereby significantly the conversion and finally the yield. During this decomposition by-products can also be formed, which can contaminate the product.

The present invention is based on the recognition that carboxylic acids under given reaction conditions in situ form reactive complexes with boron compounds of the general formula BRIR2R3, wherein the meaning of R1, R2, R3 is independently of each other -OH,-OR, -OAr group or a halogen atom, wherein the meaning of R is an aliphatic group, and Ar is an optionally substituted aromatic or heteroaromatic group containing O, N, S atoms, furthermore the meaning of R1 is an optionally substituted aromatic or aliphatic group and the meaning of R2, R3 is independently of each other as given above. The so formed complexes then react with amines resulting in acidamides as products (Figure 3).

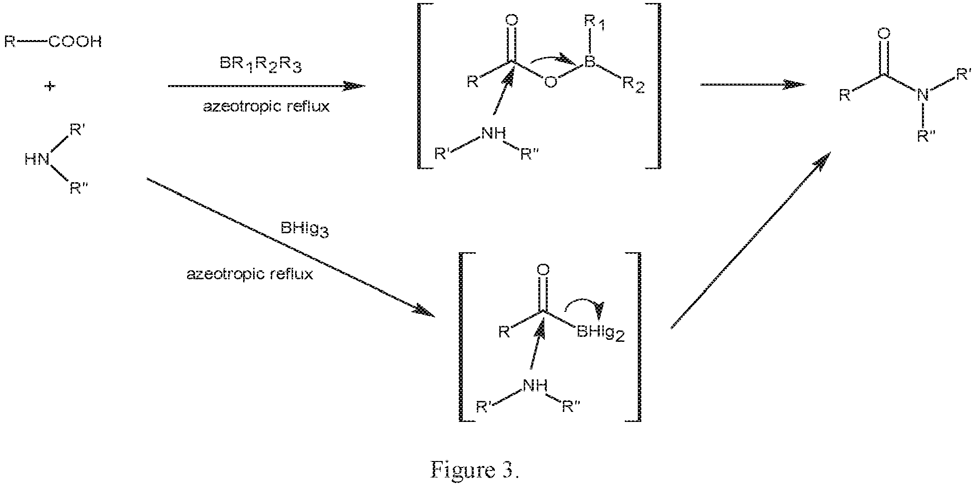

During our experiments it was found, that if 3,4-dimethoxy-phenylacetic acid of formula (III) is reacted with boric acid or with a boron compound of the general formula BR1R2R3, wherein the meaning of R1, R2, R3 is as given above - in a solvent immiscible with water such as for example toluene, an active complex of formula (VI) is formed, which can react with aminoacetaldehyde dimethyl acetal present in the reaction mixture (Figure 4).

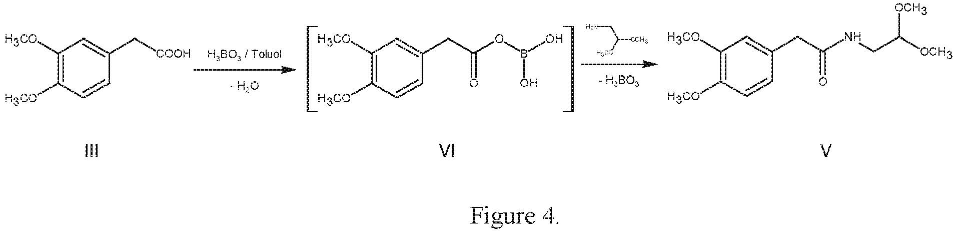

The obtained result is especially surprising even for an expert as the aminoacetaldehyde dimethyl acetal contains a dimethyl acetal group, which can be formed from an aldehyde functional group, and it is sensitive towards acids and can easily be converted into an aldehyde.

The boric acid, in spite of its acidic character, does not react with the acetal group of the aminoacetaldehyde dimethyl acetal, but only with the carboxyl group of the 3,4-dimethoxy-phenylacetic acid present in the reaction mixture. The obtained result is especially surprising, as the boric acid does not react with the acetal functional group even in that case, when the water formed in the reaction as byproduct is not removed from the reaction mixture.

The procedure of our invention does not apply strongly corrosive and toxic thionyl chloride, which is difficult to handle on an industrial scale, but a catalytic amount of a boron compound, preferably boric acid is used. A further advantage of our procedure is that during the reaction strongly aggressive and environmentally dangerous gases are not formed, therefore no further expenses arise for their absorption and neutralization. During our process hydrolytically unstable intermediates, such as acid chlorides, are not formed.

In the process worked out in our invention acid scavenger, such as e.g. N-methyl-morpholine, triethyl amine, dimethyl-aminopyridine or pyridine, is not used in the amidation step, therefore it is more economic, does not produce side-products and therefore does not burden the environment.

The process of our invention does not apply halogenated solvents, which is also more economic and does not burden the environment.

The process of our invention does not apply allergenic reagents, such as DCC, EDCI, CDI, therefore our process can be more easily applied under industrial conditions and does not require special safety regulations. Our process does not use expensive reagents, such as for example 2'-,4-ditnethoxy-6-chloro-1,3,5-triazine, therefore our process is more economical.

The process of our invention does not use reagents from which a large amount of difficultly removable side-products are formed, such as for example 2,4-dimethoxy-6-chloro-1,3,5-triazine, therefore our process does not burden the environment.

The use of boric acid is also advantageous from economical point of view because of its low price and because it is applied only in catalytic amount.

Not only the dangerous materials mentioned above can be avoided, but the conversion of the used substances, which are built into the molecule, is increased diminishing thereby the amount of the formed waste material.

In the process of our invention the ring closure of the obtained N-(2,2-dimethoxyethyl)-2-(3,4-dimethoxyphenyl)-acetamide of formula (V) is carried out in a solvent that does not contain water, preferably in methanesulfonic acid, this way the hydrolytic decomposition of the compound of formula (V) can be avoided resulting in an increased yield and the amount of the by-products considerably decreases.

In the process of our invention the yield was increased considerably, 75-85 %, as compared to that of the former processes and the purity of the obtained product remained between 99.0-99.5 % according to HPLC measurement.

The process of our invention can easily be carried out on an industrial scale from technological point of view, as the amount of the dangerous materials was decreased, there is no need to evaporate acidic compounds under diminished pressure and the reaction time was also decreased, therefore more material can be produced during the same interval. After the amidation the solvent is simply evaporated and the obtained N-(2,2-dimethoxyethyl)-2-(3,4-dimethoxyphenyl)-acetamide of formula (V) is stirred in anhydrous medium, in methanesulfonic acid at room temperature to yield 7,8-dimethoxy-1,3-dihydro-benzazepin-2-one of formula (II).

The benefit of the anhydrous medium is that the acetamide does not hydrolyze, side-products are not formed, consequently, the yield does not diminish. The methanesulfonic acid used in our process is more expensive than the acetic acid concentrated aqueous hydrochloric acid mixture, but the increased yield and the higher purity of the product compensate this to such an extent, that the material expense of the whole process is by 30 % lower.

### Boric acid catalyzed acetamide formation

The amidation reaction with boron (III) derivatives is known from the literature. In the publication Tetrahedron 2009. 65. 1085-1109, dealing with the catalysis of boron (III) compounds the authors describe the fact, that carbonic acids react with amines in the presence of different aryl-boronic acids (e.g. 3,5-trifluoromethyl-phenylboronic acid; phenylboronic acid; 3,4,5-trifluorophenylboronic acid; 3,5-bis(perfluorodecyl)phenylboronic acid) to form acidamides under conditions, when no alcohols are present in the reaction mixture. In the presence of alcohols esters are formed. In their publication they mention, that boric acid is also applied in the synthesis of some drug substance such as for example Felcanide, Repaglinide and Alfuzosin. These reactions are carried out in the presence of 10 mol% boric acid in a solvent immiscible with water, such as toluene or o-xylene.

In the aforementioned publication exclusively such amines are used for the formation of acid amides, which do not contain groups sensitive towards acid hydrolysis - as e.g. the acetal group.

In the experimental part of Organic Syntheses 2005. 81. 262-272. also the boric acid catalyzed amide formation is described. But in this reaction benzylamine is used as amine component, which is not sensitive towards acidic hydrolysis, and in the given examples neither can be found an acid labile amine. In this reaction 1 mol% of the boric acid is used, and the solvent is toluene.

The process of our invention is therefore surprising for an expert, as we did not find in the literature an acetamide formation reaction, which would use aminoacetaldehyde dimethyl acetal as starting material and a boron compound of the general formula BR1R2R3, wherein the meaning of R1, R2, R3 is independently of each other -OH, -OR, -OAr group or a halogen atom, furthermore wherein R1 is an optionally substituted aromatic or aliphatic group and R2, R3 are independently of each other -OH, -OR, -OAr groups or a halogen, as for example boric acid as catalyst.

A broad variety of boric acids is commercially available.

### Ring-closure

According to literature data the ring-closure of the obtained N-(2,2-dimethoxyethyl)-2-(3,4-dimethoxyphenyl)-acetamide of formula (V) is usually carried out in acetic acid with aqueous concentrated hydrochloric acid. The presence of water in the reaction is disadvantageous, as during the processes used for the formation of the acetamide usually acid traces remain in the reaction mixture, which trigger a hydrolytic decomposition process of the aminoacetaldehyde dimethyl acetal, diminishing thereby the yield and contaminating the obtained product.

In the process of our invention we recognized, that the presence of water in the ring-closure reaction is not desirable, therefore we looked for a compound with Lewis acid properties, which can be applied on an industrial scale as well. In the process worked out in our invention methanesulfonic acid is applied, which is a widely used compound of Lewis acid character and can be used as solvent too. The advantage of it is that it is cheaper than the above mentioned trifluoromethanesulfonic acid (the price ratio is 1:20), therefore the expenditures are lower on an industrial scale.

If the ring-closure reaction is carried out with methanesulfonic acid, surprisingly even for a skilled in the art, there is no or only a very little amount of byproduct formed, the purity of the obtained product without further purification remains between 99.0-99.5 % according to HPLC. The product can be isolated from the reaction mixture in a yield of 75-85%, which is also a surprising increase as compared to the earlier yields, which remained below 60% and it is an economically very important fact.

According to our investigations the ring-closure reaction can be carried out so effectively not only in methanesulfonic acid, but for instance in anhydrous acetic acid, containing hydrogen bromide. But in this case brominated by-products are also formed because of the good halogenating properties of the hydrogen bromide, decreasing this way the purity of the product.
In summary, the advantages of the present invention are the following:
During the synthesis of N-(2,2-dimethoxyethyl)-2-(3,4-dimethoxyphenyl)-acetamide of formula (V)
- we do not apply chemical materials which are difficult to handle, dangerous and require special safety treatments.
- no strongly corrosive gases or other by-products are formed, which would harm the environment, only water is formed during the reaction.
- an environmentally friendly catalyst is applied and even that only in catalytic amounts.
- we do not apply chlorinated solvents.
- the reaction is simpler from technological point of view, as only boiling is required at reflux temperature with a simultaneous removal of water.
- we do not produce an intermediate, which is difficult to handle and decomposes by hydrolysis, such as an acid chloride, therefore no environmental or safety hazards arise.
- the industrial equipments are not exposed to extreme corrosive conditions, which means a long-lasting economic benefit
- during the ring-closure anhydrous conditions are used resulting in an increase of the yield by 25-40 %, this way a yield of 75-85 % could be obtained
- the HPLC purity of the product obtained in the ring-closing reaction remains in the range of 99.0-99.5 % without purification.
- with the process developed in our invention a material expense reduction of 30 % can be achieved as compared to the processes known from the literature.

### Detailed description of the invention

The invention relates to a process for the synthesis of 7,8-dimethoxy-1,3-dihydro-benzazepin-2-one of formula (II), which is a key-intermediate in the production of pharmaceutically applicable 3-(3-{[((7S)-3,4-dimethoxybicyclo[4,2,0]octa-1,3,5-trien-7-yl)-methyl]-methylamino}-propyl)-1,3,4,5-tetrahydro-7,8-dimethoxy-2H-3-benzazepin-2-one (also known as Ivabradin) of formula (I). According to the process 3,4-dimethoxy-phenylacetic acid of formula (III) is reacted with aminoacetaldehyde dimethyl acetal in a solvent immiscible with water with a boron compound of the general formula BR1R2R3, which is described above in detail, preferably in the presence of boric acid, and the obtained N-(2,2-dimethoxyethyl)-2-(3,4-dimethoxyphenyl)-acetamide of formula (V) is cyclized with an anhydrous acid of Lewis character, selected from methanesulfonic acid or hydrogen bromide in acetic acid, preferably methanesulfonic acid.

The 7,8-dimethoxy-1,3-dihydro-benzazepin-2-one of formula (II) is synthesized by reacting 3,4-dimethoxy-phenylacetic acid of formula (III) with aminoacetaldehyde dimethyl acetal in a solvent immiscible with water, as e.g. xylene or toluene, preferably toluene. The amount of the organic solvent used for the amidation reaction is 2-15 fold calculated on the amount of 3,4-dimethoxy-phenylacetic acid of formula (III), preferably 2.5 fold.

In the process according to our invention the catalyst used for the amidation reaction is a boron compound of the general formula BR1R2R3, preferably boric acid, wherein the meaning of R1, R2, R3 is independently from each other -OH, -OR, -OAr group or a halogen atom, wherein the meaning of R is an aliphatic group, and Ar is an optionally substituted aromatic hydrocarbon or a heteroaromatic group, containing O, N, S atoms, furthermore wherein the meaning of R1 is an optionally substituted aromatic or aliphatic group and the meaning of R2, R3 is independently of each other the same as above.

The defined functional groups in the meaning of R1, R2, R3 substituents can be the same, or can be different, consequently in the boron compound used as catalyst different variations of them can be applied, e.g. the compounds of the general formula BOH (OR)2 or the phenylboronic acid and its derivatives of the general formula PhB(OR)2, e.g. 3,4,5-trifluorophenyl boronic acid, 3,4-difluorophenyl boronic acid, aminophenyl boronic acid, 3,5-bis (perfluorodecyl)-phenylboronic acid, etc.

The aliphatic groups in the meaning of R1 and R substituents can be the same, or can be different as well, e.g. B(OCH3)3 or BOCH3(OC2H5)2. The aromatic groups can also be the same or can be different, e.g. B(OPh)3 or BOPh(O-(subtituted-Ph)2.

The optionally substituted aromatic groups in the meaning of R1 and Ar can represent, but not limited to such mono- or bicyclic aryl- or heteroaryl group which in given case can be substituted with one or more, preferably with 1-4 substituents, e.g. with halogen atoms, alkyl- or alkoxy groups, or an aryl group. The optionally substituted aromatic group can be for example a group containing 6-18 carbon atoms, preferably phenyl or naphthyl group.

The aliphatic group might be straight or branched chain or cyclic group, which optionally can be substituted with one or more identical or different heteroatoms. Preferably the straight or branched chain 1-18 carbon atom containing groups can be for example ethyl, propyl, isopropyl, tert-butyl, isopentyl, octyl, decyl group, and the cycloalkyl groups can be for example cyclohexyl group.

The boron catalyst of the present invention is commercially available or can be prepared by known methods obvious for an expert.

The boric acid esters used in the present invention can be practically prepared by condensation reaction of boric acid or boronic acids and a suitable alcohol or a mixture of alcohols under the reaction conditions.

In the present invention the amount of the boron (III) catalyst can vary between 5-100 mol%, preferably it can be 10 mol%.

The temperature of the amide formation reaction according to the present invention is room temperature, preferably it can vary between 25°C and the boiling point of the applied solvent immiscible with water.

According to the process of the present invention an anhydrous Lewis type acid is used for the ring closure reaction, which is selected from hydrogen bromide in acetic acid or methanesulfonic acid, preferably methanesulfonic acid.

The amount of the anhydrous acid used in the ring closure reaction according to the present invention is 3-15 fold as compared to the amount of 3,4-dimethoxy-phenylacetic acid of formula (III), preferably it is 7-fold.

According to the present invention the temperature of the ring closure reaction can vary between 20-50°C, preferably between 20-25°C.

According to the present invention the yield of the formed 7,8-dimethoxy-1,3-dihydro-benzazepin-2-one of formula (II) can vary between 70-90 %, typically between 75-85 %.

The HPLC purity of the 7,8-dimethoxy-1,3-dihydro-benzazepin-2-one of formula (II) obtained according to the process described in the present invention is between 98-100 %, typically between 99.0-99.5 %.

The intermediate of formula (II) can be converted into the compound of formula (I) by known methods, for example via the reaction depicted below.

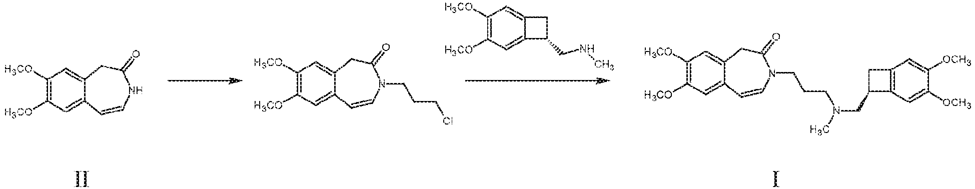

Further details of the present invention are illustrated by the following not limiting examples.

### Example 1

### Synthesis of N-(2,2-dimethoxyethyl)-2-(3,4-dimethoxyphenyl)-acetamide of formula (V) with boric acid.

A stirred mixture of 10.0 g (0.05 mol) of 3,4-dimethoxy-phenylacetic acid, 25.0 ml of toluene, 5.89 g (0.056 mol) of aminoacetaldehyde dimethyl acetal and 0.31 g (0.005 mol) of boric acid was warmed to reflux temperature and stirring was continued at this temperature for 5 h. The water formed in the reaction was continuously removed. After completion of the reaction the reaction mixture was concentrated.
The weight of the obtained product was 14.66 g.

### Example 2

### Synthesis of 7,8-dimethoxy-1,3-dihydro-benzazepin-2-one of formula (II)

70.0 ml of methanesulfonic acid was given to the residue obtained in Example 1 and the reaction mixture was stirred at 20-25°C for 5 h. After completion of the reaction the mixture was poured into 300 ml of water. The obtained slurry was stirred for 30 min, filtered, washed until neutrality and dried.

The weight of the obtained 7,8-dimethoxy-1,3-dihydro-benzazepin-2-one was 8.71 g (yield 77.9 %, calculated on 3,4-dimethoxy-phenylacetic acid) (HPLC: 99.34 %)

### Example 3

### Synthesis of N-(2,2-dimethoxyethyl)-2-(3,4-dimethoxyphenyl)-acetamide of formula (V) with phenylboronic acid

A stirred mixture of 2.0 g (0.01 mol) of 3,4-dimethoxy-phenylacetic acid, 5.0 ml of toluene, 1.18 g (0.011 mol) of aminoacetaldehyde dimethyl acetal and 0.12 g (0.001 mol) of phenylboronic acid was warmed to reflux temperature and stirring was continued at this temperature for 2 h. The water formed in the reaction was continuously removed. After completion of the reaction the mixture was concentrated.

The weight of the obtained product was 3.0 g.

### Example 4

### Synthesis of N-(2,2-diniethoxyethyl)-2-(3,4-dimethoxyphenyl)-acetamide of formula (V) with tributyl borate

A stirred mixture of 1.0 g (5 mmol) of 3,4-dimethoxy-phenylacetic acid, 2.5 ml of toluene, 0.59 g (5.5 mmol) of aminoacetaldehyde dimetylacetal and 0.115 g (0.5 mmol) of tributyl borate was warmed to reflux temperature and stirring was continued at this temperature for 2 h. After completion of the reaction the mixture was concentrated under diminished pressure.

The weight of the obtained product was 1.63 g.

### Example 5

### Synthesis of N-(2,2-dimethoxyethyl)-2-(3,4-dimethoxyphenyl)-acetamide of formula (V) with boron trifluoride diethyletherate

A stirred mixture of 1.0 g (5 mmol) of 3,4-dimethoxy-phenylacetic acid, 2.5 ml of toluene, 0.59 g (5.5 mmol) of aminoacetaldehyde dimethyl acetal and 0.07 g (0.5 mmol) of boron trifluoride diethyletherate was warmed to reflux temperature and stirring was continued at this temperature for 2 h. The water formed in the reaction was continuously removed. After completion of the reaction the mixture was concentrated under diminished pressure.
The weight of the obtained product was 1.69 g.

## Claims

1. Process for the synthesis 7,8-dimethoxy-1,3-dihydro-benzazepin-2-one of formula (II) from 3,4-dimethoxy-phenylacetic acid of formula (III) 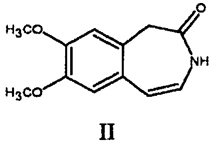 ***characterized by***
i) reacting the compound of formula (III) with aminoacetaldehyde dimethyl acetal of formula (IV) in an organic solvent immiscible with water 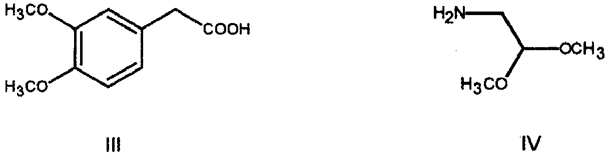 in the presence of catalytic amount of boron compound of formula **BR1R2R3,** wherein the meaning of R1, R2, R3 is independently of each other -OH, -OR, -OAr group or a halogen atom, wherein the meaning of R is an aliphatic group, and Ar is an optionally substituted aromatic or heteroaromatic group containing O, N, S atoms, furthermore the meaning of R1 is an optionally
substituted aromatic or aliphatic group and the meaning of R2, R3 is independently of each other as given above;
ii) then reacting the obtained N-(2,2-dimethoxyethyl)-2-(3,4-dimethoxyphenyl)-acetamide of formula (V) 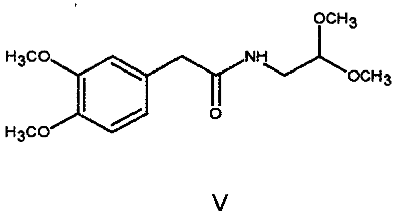 with an anhydrous acid to furnish the compound of formula (II), wherein the anhydrous acid is methanesulfonic acid or hydrogen bromide in acetic acid.

2. The process according to claim 1, wherein the compound of formula **BR₁R₂R₃** used in the amide formation reaction is boric acid, phenylboronic acid, tributyl borate or boron trifluoride diethyletherate, preferably boric acid.

3. The process according to claim 2, wherein the boron catalyst is used in an amount of 5-100 mol%.

4. The process according to claim 3, wherein the boron catalyst is used in an amount of 10 mol%.

5. The process according to claim 1, wherein the solvent immiscible with water used in the amide formation reaction is toluene or xylene, preferably toluene.

6. The process according to claim 1, wherein the water formed in the amidation reaction is removed continuously.

7. The process according to claim 1, wherein the amide formation reaction is carried out at a temperature between 25°C and the boiling point of the applied solvent.

8. The process according to claim 1, wherein the anhydrous acid used in the ring-closure reaction is methanesulfonic acid.

9. The process according to claim 1, wherein the amount of the anhydrous acid used in the ring-closure reaction is 3-15 fold as compared to the amount of 3,4-dimethoxy-phenylacetic acid of formula (III), preferably 7 fold.

10. The process according to claim 1, wherein the ring-closure reaction is carried out at a temperature between 20-50 °C, preferably between 20-25°C.

11. Process according to claim 1 for the synthesis of a compound of formula (I) or a pharmaceutically acceptable salt thereof 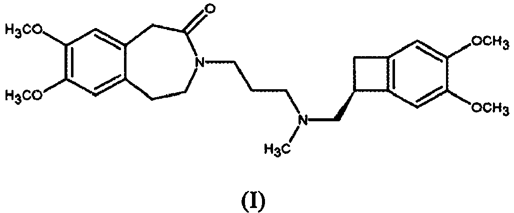
comprising, after i) and ii):
iii) transforming the obtained compound of formula (II) into the compound of formula (I) or an acid addition salt thereof by known methods.

## Patentansprüche

1. Verfahren zur Synthese von 7,8-Dimethoxy-1,3-dihydro-benzazepin-2-on der Formel (II) aus 3,4-Dimethoxyphenylessigsäure der Formel (III) 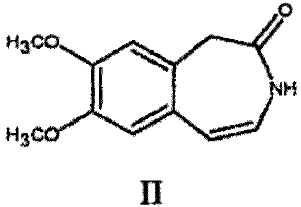 **dadurch gekennzeichnet, dass**
i) die Verbindung der Formel (III) mit Aminoacetaldehyddimethylacetal der Formel (IV) in einem organischen Lösungsmittel, das mit Wasser nicht mischbar ist, 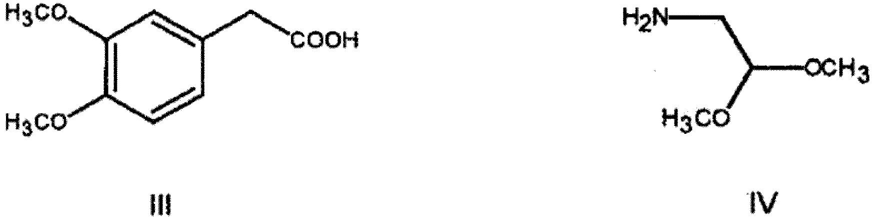 in Gegenwart einer katalytischen Menge von einer Borverbindung der Formel **BR1R2R3** umgesetzt wird, wobei die Bedeutung von R1, R2, R3 unabhängig voneinander eine Gruppe -OH, -OR, -OAr oder ein Halogenatom ist, worin die Bedeutung von R eine aliphatische Gruppe ist und
Ar eine optional substituierte aromatische oder heteroaromatische Gruppe ist, die 0-, N-, S-Atome enthält, zusätzlich die Bedeutung von R1 eine optional substituierte aromatische oder aliphatische Gruppe ist und die Bedeutung von R2, R3 ist unabhängig voneinander wie oben angegeben ist;
ii) dann das erhaltene N-(2,2-Dimethoxyethyl)-2-(3,4-dimethoxyphenyl)acetamid der Formel (V) 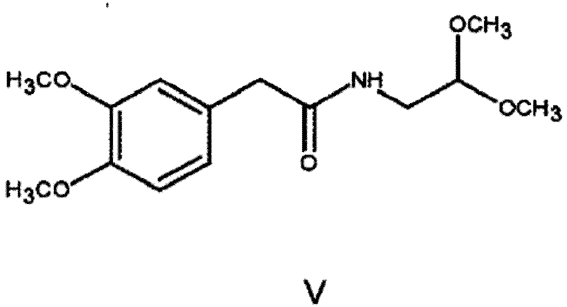 mit einer wasserfreien Säure umgesetzt wird, um die Verbindung der Formel (II) bereitzustellen, wobei die wasserfreie Säure Methansulfonsäure oder Bromwasserstoff in Essigsäure ist.

2. Verfahren gemäß Anspruch 1, bei dem die Verbindung der Formel **BR1R2R3,** die in der Amidbildungsreaktion verwendet wird, Borsäure, Phenylboronsäure, Tributylborat oder Bortrifluorid-Diethyletherat, bevorzugt Borsäure, ist.

3. Verfahren gemäß Anspruch 2, bei dem der Borkatalysator in einer Menge von 5 bis 100 Mol-% verwendet wird.

4. Verfahren gemäß Anspruch 3, bei dem der Bor-Katalysator in einer Menge von 10 Mol-% verwendet wird.

5. Verfahren gemäß Anspruch 1, bei dem das Lösungsmittel, das mit Wasser nicht mischbar ist, das in der Amidbildungsreaktion verwendet wird, Toluol oder Xylol, bevorzugt Toluol, ist.

6. Verfahren gemäß Anspruch 1, bei dem das in der Amidbildungsreaktion gebildete Wasser kontinuierlich entfernt wird.

7. Verfahren gemäß Anspruch 1, bei dem die Amidbildungsreaktion bei einer Temperatur zwischen 25°C und dem Siedepunkt des eingesetzten Lösungsmittels durchgeführt wird.

8. Verfahren gemäß Anspruch 1, bei dem die wasserfreie Säure, die in der Ringschlussreaktion verwendet wird, Methansulfonsäure ist.

9. Verfahren gemäß Anspruch 1, bei dem die Menge der wasserfreien Säure, die in der Ringschlussreaktion verwendet wird, im Vergleich zu der Menge an 3,4-Dimethoxyphenylessigsäure der Formel (III) die 3- bis 15-fache, bevorzugt die 7-fache, Menge ist.

10. Verfahren gemäß Anspruch 1, bei dem die Ringschlussreaktion bei einer Temperatur von 20 bis 50°C, bevorzugt von 20 bis 25°C, durchgeführt wird.

11. Verfahren gemäß Anspruch 1 zur Synthese einer Verbindung der Formel (I) oder eines pharmazeutisch akzeptablen Salzes davon 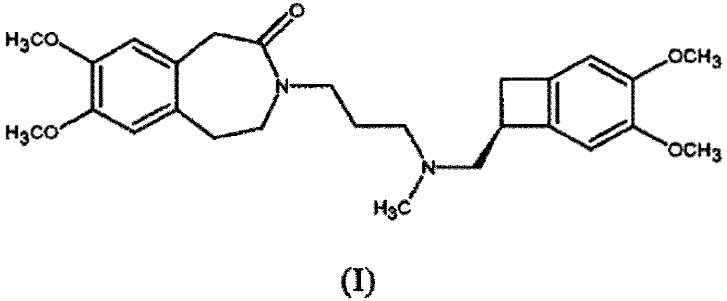
umfassend nach i) und ii):
iii) das Überführen der erhaltenen Verbindung der Formel (II) in die Verbindung der Formel (I) oder eines Säureadditionssalzes davon durch bekannte Verfahren.

## Revendications

1. Procédé pour la synthèse de 7,8-diméthoxy-,3-dihydro-benzazépin-2-one de formule (II) à partir d'acide 3,4-diméthoxy-phénylacétique de formule (III) 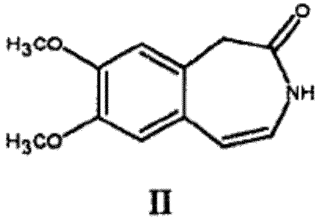 ***caractérisé par***
i) la réaction du composé de formule (III) avec du diméthyl-acétal d'aminoacétaldéhyde de formule (IV) dans un solvant organique non miscible avec l'eau 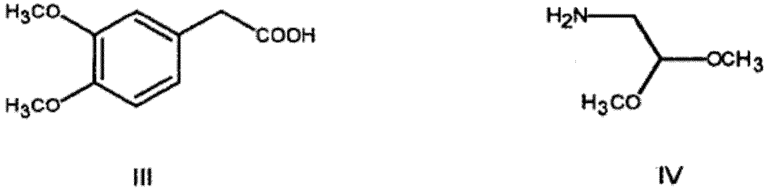 en présence d'une quantité catalytique de composé de bore de formule **BR1R2R3,** dans lequel R1, R2, R3 désignent indépendamment les uns des autres un groupe -OH,-OR, -OAr ou un atome d'halogène, dans lequel R désigne un groupe aliphatique, et Ar est un groupe aromatique ou hétéro-aromatique éventuellement substitué contenant des atomes de O, N, S, en outre, R1 désigne un groupe aromatique ou aliphatique éventuellement substitué et R2 et R3 ont indépendamment l'un de l'autre la même signification que ci-dessus ;
ii) puis la réaction du N-(2,2-diméthoxyéthyl)-2-(3,4-diméthoxyphényl)-acétamide de formule (V) obtenu 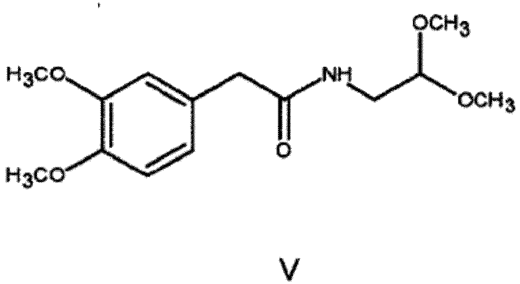 avec un acide anhydre pour fournir le composé de formule (II), dans lequel l'acide anhydre est l'acide méthane-sulfonique ou du bromure d'hydrogène dans de l'acide acétique.

2. Procédé selon la revendication 1, dans lequel le composé de formule **BR₁R₂R₃** utilisé dans la réaction de formation d'amide est l'acide borique, l'acide phénylboronique, le borate de tributyle ou le diéthyléthérate de trifluorure de bore, de préférence l'acide borique.

3. Procédé selon la revendication 2, dans lequel le catalyseur bore est utilisé en une quantité de 5 à 100 % en mole.

4. Procédé selon la revendication 3, dans lequel le catalyseur bore est utilisé en une quantité de 10 % en mole.

5. Procédé selon la revendication 1, dans lequel le solvant non miscible avec l'eau utilisé dans la réaction de formation d'amide est le toluène ou le xylène, de préférence le toluène.

6. Procédé selon la revendication 1, dans lequel l'eau formée dans la réaction d'amidation est éliminée en continu.

7. Procédé selon la revendication 1, dans lequel la réaction de formation d'amide est réalisée à une température entre 25 °C et le point d'ébullition du solvant appliqué.

8. Procédé selon la revendication 1, dans lequel l'acide anhydre utilisé dans la réaction de fermeture de cycle est l'acide méthane-sulfonique.

9. Procédé selon la revendication 1, dans lequel la quantité d'acide anhydre utilisé dans la réaction de fermeture de cycle est de facteur 3 à 15 par rapport à la quantité d'acide 3,4-diméthoxy-phénylacétique de formule (III), de préférence de facteur 7.

10. Procédé selon la revendication 1, dans lequel la réaction de fermeture de cycle est réalisée à une température entre 20 et 50 °C, de préférence entre 20 et 25 °C.

11. Procédé selon la revendication 1 pour la synthèse d'un composé de formule (I) ou d'un sel pharmaceutiquement acceptable de celui-ci 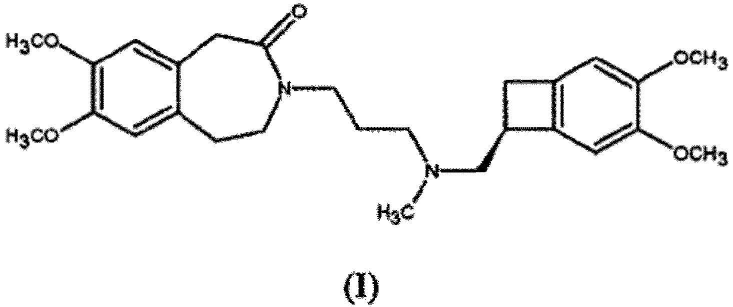
comprenant, après les étapes i) et ii) :
iii) la transformation du composé de formule (II) obtenu en composé de formule (I) ou en un sel d'addition acide de celui-ci par des procédés connus.
